# DEMANDE DE BREVET EUROPEEN

(11) **EP 0 527 070 A1**
(43) Date de publication de la demande: **10.02.1993**
(21) Numéro de dépôt: 92401941.7
(22) Date de dépôt: 06.07.1992
(51) Int. Cl.: C12N 1/14, C12Q 1/68, C12Q 1/04

(54) **Souches de Penicillium caseicolum fromagères et "antimucor"**

(30) Priorité: 05.07.1991 FR 9108495; 05.07.1991 FR 9108496
(71) Demandeur: SYSTEMS BIO-INDUSTRIES, F-92100 Boulogne (FR)
(72) Inventeur: Bouvier, Isabelle, 91940 Les Ullis (FR); Brygoo, Yves, 78470 Saint Remy Les Chevreuse (FR); Lima, Oscar, 91000 Evry (FR)
(74) Mandataire: Gillard, Marie-Louise

(57) **Abrégé**

La présente invention a pour objet de nouvelles souches de Penicillium caseicolum, qui peuvent être obtenues à partir d'une nouvelle souche de Penicillium caseicolum déposée à la CNCM sous le n° I-1119, et qui ont des aptitudes fromagères. Elle a également pour objet un procédé pour l'obtention de ces souches, qui consiste à :
- étaler des spores de la souche CNCM I-1119 sur un milieu haploïdisant;
- repiquer les colonies apparues sur un milieu minimum;
- sélectionner, parmi les souches obtenues, celles qui présentent une activité "antimucor";
- isoler parmi ces dernières celles qui présentent un profil de restriction pour le couple enzyme Hind III-sonde d'ADN déposée à la CNCM sous le n° I-1120, identique à l'un des profils B′ ou C.

Application : Fabrication de fromages.

## Description

La présente invention a pour objet de nouvelles souches de Penicillium caseicolum "antimucor", utiles dans l'industrie fromagère, notamment pour la fabrication des fromages à croûte fleurie. Elle a également pour objet un procédé pour l'obtention de ces souches ainsi que la sonde d'ADN permettant de les identifier.

Dans la présente description on désigne par "fromages à croûte fleurie" les fromages présentant une croûte formée par le développement de champignons, principalement de l'espèce Penicillium caseicolum. Ces champignons de surface ont un rôle important dans l'affinage de la pâte du fromage qu'ils recouvrent grâce à leur équipement enzymatique (protéases et lipases).

Les fromages à croûte fleurie sont fabriqués industriellement en particulier par ensemencement des caillés avec des spores de champignons appartenant à l'espèce Pénicillium caseicolum. Un des problèmes majeurs rencontrés lors de cette fabrication est le développement de moisissures indésirables sous forme d'un tapis mycélien appelé "poil de chat". Cet accident de fabrication est dû à différentes espèces de champignons appartenant toutes au genre Mucor (M. globosus, M. hiemalis, M. racemosus, M. mucedo). Ces espèces contaminantes se caractérisent par leur croissance très rapide qui leur permet d'envahir en quelques jours les fromages en cours de fabrication et de contaminer les locaux de fabrication. Ce type d'accident a lieu plus particulièrement pendant les périodes où l'humidité relative est la plus importante.

Les types de fromages les plus touchés sont les fromages à croûte fleurie, tels que notamment les camemberts, les fromages à pâtes stabilisées, les bries et les chèvres.

Pour lutter contre ces contaminations, divers moyens peuvent être mis en oeuvre lors d'une fabrication ; ces moyens sont soit des méthodes physiques ou chimiques, telles que :
- diminution de l'humidité relative des hâloirs (HR<85 %) ;
- augmentation de la teneur en sel des fromages (NaCl>1,5 %) ; soit des méthodes microbiologiques, telles que :
   - ensemencement de P. caseicolum dans le lait d'emprésurage pour un démarrage de la croissance plus précoce ;
   - ensemencement plus dense pour une couverture du caillé plus rapide ;
   - choix de souches à développement plus rapide ; et
   - enfin, utilisation de souches de P. caseicolum particulières, dites "antimucor".

La particularité de ces souches "antimucor" est leur potentialité à inhiber le développement de différentes espèces du genre Mucor.

Les souches de P. caseicolum possédant cette aptitude sont peu nombreuses et, parmi elles, seul un nombre restreint est utilisable en fromagerie. En effet, ces souches, outre le caractère "antimucor" avantageux, présentent de nombreux défauts :
- une teinte jaune du thalle,
- un développement plus lent,
- un mycélium dense donnant souvent un aspect cartonneux à la croûte du fromage,
- une activité protéolytique partielle conduisant souvent à des défauts d'amertume,
- une mauvaise sporulation limitant la commercialisation de certaines d'entre elles.

Afin de rendre possible l'utilisation de ces souches en masquant au mieux leurs désavantages, les fabricants de ferments proposent, sous le label "souche antimucor", des mélanges de souches dans lesquels sont associées une souche "antimucor" et une ou plusieurs souches présentant de bonnes aptitudes fromagères. Les proportions de chaque souche sont variables suivant l'effet recherché ou l'utilisation souhaitée.

Parmi les souches "antimucor" disponibles dans le commerce on peut citer par exemple les souches PSM1, PSM2 et SAM2, commercialisées par les Laboratoires GRANDAY-ROGER et LACTOLABO.

On notera en fait que la pratique la plus courante consiste à utiliser le mélange "antimucor" en association avec la ou les souches habituellement mises en oeuvre pour la fabrication concernée. Ceci a bien sûr pour conséquence de diminuer l'efficacité du pouvoir inhibiteur vis-à-vis des Mucor contaminants et d'entraîner une irrégularité dans la fabrication du fait de l'hétérogénéïté des mélanges de souches.

Les nouvelles souches selon la présente invention présentent des caractéristiques avantageuses par rapport aux "souches antimucor" traditionnelles. En effet, elles cumulent des aptitudes habituellement portées par des souches différentes : activité "antimucor" et aptitudes fromagères.

Les souches selon l'invention sont caractérisées par un profil de restriction particulier pour le couple enzyme Hind III-sonde d'ADN déposée dans des phages auprès de la Collection Nationale de Cultures de Microorganismes (CNCM) (Institut Pasteur, Paris) sous le n° I-1120. Cette sonde sera dénommée, ci-après, sonde d'ADN CNCM I-1120. Ce profil de restriction est soit le profil B′, soit le profil C.

Le profil de restriction B′ est caractérisé par 5 fragments de restriction : 1,2 kb; 2,9 kb; 4,1 kb; 4,8 kb; et 5,4 kb; le quatrième fragment étant moins abondant que pour les champignons connus.

Le profil de restriction C est caractérisé par 9 fragments de restriction : 1,2 kb; 2,2 kb; 2,4 kb; 2,9 kb; 4,1 kb; 4,3 kb; 4,8 kb; 5,4 kb; et 5,9 kb.

Les souches de Penicillium caseicolum de l'invention sont de couleur blanche, ce qui est particulièrement souhaitable pour l'utilisation fromagère.

Les souches préférées selon l'invention sont celles caractérisées par le profil de restriction C.

Elles peuvent être obtenues à partir d'une nouvelle souche de Penicillium caseicolum, qui a été déposée à la Collection Nationale de Culture de Microorganismes, Institut Pasteur, PARIS, (CNCM) sous le numéro I-1119.

Les dépôts des souches ci-dessus, CNCM I-1119 et CNCM I-1120 ont été effectués conformément au Traité de Budapest le 18 Juin 1991.

La souche de Penicillium caseicolum CNCM I-1119 provient de la fusion de protoplastes de deux souches de Penicillium, dont l'une est "antimucor".

Le procédé d'obtention des souches selon l'invention à partir de la souche CNCM 1-1119 consiste à :
1) étaler des spores de la souche CNCM I-1119 sur un milieu haploïdisant ;
2) repiquer les colonies apparues sur un milieu minimum ;
3) sélectionner parmi les souches obtenues celles qui présentent une activité "antimucor", et qui ont un thalle de couleur blanche ;
4) isoler parmi ces dernières celles qui présentent un profil de restriction pour le couple enzyme Hind III-sonde d'ADN déposée à la CNCM sous le n° I-1120, identique à l'un des profils B′ ou C.

La première étape du procédé peut être réalisée sur différents milieux complets contenant un agent haploïdisant quelconque.

A titre d'exemples de milieux complets, on peut utiliser des milieux à base d'extraits de pommes de terre ou d'extraits de malt, notamment le milieu PDA (Potato-Dextrose-Agar) commercialisé par DIFCO Laboratories sous la référence 0013-01-4 ou le milieu malt acide appelé "milieu B" par de FALANDRE et al., dans Appln. Environ. Microbiol., 53 : 1500-1503, (1987).

A titre d'agent haploïdisant approprié on peut citer notamment la p-fluorophénylalanine [LHOAS (1961) Nature 190 : 744] et le bénomyl [HASTIE (1970) Nature 226 : 77].

L'agent haploïdisant doit être utilisé à une concentration qui inhibe la croissance et la formation des colonies. Cette concentration varie selon la sensibilité des souches et il appartiendra à l'homme de l'art de définir par des tests de routine classiques la concentration convenable pour chaque souche.

Toutefois, on indiquera que des concentrations en agent haploïdisant comprises entre 0,5 ppm et 0,04 ppm sont appropriées aux fins de l'invention.

Le temps de contact avec l'agent haploïdisant peut varier entre 3 et 8 jours suivant la vitesse de développement du microorganisme.

Pour la souche CNCM I-1119, cette étape est avantageusement réalisée sur le milieu malt acide (pH 5) (= milieu B décrit par de Falandre et al. dans l'article cité ci-dessus), contenant 0,06 ppm de bénomyl. Le bénomyl est la substance active du benlate, produit commercialisé par Du Pont de Nemours.

Des spores de la souche CNCM I-1119 sont maintenues au contact de l'agent haploïdisant (milieu malt acide + benlate), pendant 8 jours maximum à une température de 23°C et sous une hygrométrie de 90 à 95%.

La seconde étape du procédé consiste à repiquer les colonies apparues sur le milieu halpoïdisant en les bouturant sur un milieu minimum, par exemple, le milieu minimum décrit par BOUVIER J. (1967) C.R. Acad. Sci. Ser. D 265 : 1305-1308.

La troisième étape consiste à tester l'activité inhibitrice des souches purifiées retenues vis-à-vis du Mucor. Cette activité inhibitrice peut être déterminée selon le test de confrontations de souches définies ci-après.

Le test est effectué sur des boîtes de Petri de 10 cm de diamètre contenant 20 ml de milieu malt acide.

Il consiste à déposer 10⁵ spores d'une souche de Mucor sur un diamètre de la boîte de Petri à l'aide d'une pipette effilée.

Les souches de Penicillium caseicolum à tester sont disposées, sous forme d'implants allongés de 10 mm x 1,5 mm, sur deux lignes situées de part et d'autre, à 3 cm de distance de la culture de Mucor.

Les conditions donnant la réponse optimale sont : la culture d'une souche de Mucor globosus, l'utilisation du milieu malt acide et le dépôt des implants de Penicillium caseicolum 24 heures après l'ensemencement du Mucor. Après 4 jours d'incubation à 23°C, une zone de non croissance du Mucor en face de l'implant de Penicillium permet de caractériser une souche inhibitrice dite "antimucor" ou résistante au Mucor. Si l'on prolonge la culture 4 jours supplémentaires, le Penicillium pousse au-dessus du Mucor.

Une souche est non-inhibitrice ou sensible au Mucor, lorsque le Mucor arrive au contact du Penicillium après 4 jours de culture.

A ce niveau, chaque souche "antimucor" est purifiée par isolement monospore. Les souches retenues sont conservées sous azote liquide et sous huile de vaseline.

Il est avantageux de répéter une ou plusieurs fois les étapes 1), 2) et 3) sur les souches "antimucor" sélectionnées à l'étape 3).

Au cours de la quatrième étape, on sélectionne ensuite, parmi les souches "antimucor" obtenues, celles dont l'ADN génomique digéré par l'enzyme de restriction Hind III révèle, par hybridation avec la sonde d'ADN déposée à la CNCM sous le n° I-1120, l'un des profils B′ ou C.

Cette sélection est basée sur les connaissances suivantes :
L'enchaînement des quatre bases constitutives de l'ADN (bases A, C, G et T) peut générer certaines petites séquences nucléotidiques présentant la particularité d'être reconnues par des enzymes qui coupent, ou qui restreignent l'ADN en ces points précis. L'ADN génomique coupé par une enzyme de restriction génère des fragments de tailles variables. On peut séparer ces fragments de restriction en fonction de leur taille grâce à une électrophorèse sur gel d'agarose, où l'ADN est coloré par le bromure d'éthidium (B.E.T.). Les profils de restriction ainsi obtenus sont propres à une souche donnée et à une enzyme donnée.

Une souche peut différer d'une autre par une modification d'une base intervenue dans un site de restriction. La méthode PLFR (polymorphisme de la longueur des fragments de restriction) consiste à mettre en évidence ce type de modifications. En effet, un site de restriction muté n'est plus coupé par son enzyme, ceci entraîne une variation dans le profil de restriction. Il suffit donc de mettre en évidence ces variations pour différencier ces deux souches.

Cette quatrième étape consiste à réaliser un Southern BLOT avec les différentes souches de Penicillium caseicolum, puis une hybridation ADN-ADN avec la sonde d'ADN CNCM I-1120, selon le mode opératoire décrit par Sambrook et al (Molecular Cloning, Cold Spring Harbor Laboratory Press 1989). Ce protocole est avantageusement réalisé en préférant les étapes suivantes :
- l'ADN génomique des différentes souches de Penicillium caseicolum est préparé selon Daboussi et al (Current Genetics, 15: 453-456, 1989), puis restreint par l'enzyme Hind III;
- ces ADN, après migration électrophorétique (Agarose 0,8% ; 24h., sous 1V/cm en tampon T.A.E. (tris-Acide acétique-EDTA) 1X, sont transférés sous vide (Vacum Gene XL Bloting System ; Pharmacia) sur une membrane de nylon (Hybond N ; Amersham) ;
- l'ADN de la sonde CNCM I-1120 est radiomarquée au ³²P (Random Primed DNA Labeling Kit ; Boehringer Mannheim), puis les hybridations sont menées à 65°C.

Les souches selon l'invention présentent des aptitudes technologiques au moins équivalentes aux souches couramment mises en oeuvre en fabrications fromagères ; ces aptitudes sont la croissance, la hauteur du mycélium, la densité du mycélium, les activités protéolytiques et lipolytiques, le comportement sur tranches de caillés. Les protocoles de détermination de ces aptitudes sont indiqués dans les exemples ci-après.

L'invention va être maintenant décrite plus en détail par les exemples illustratifs ci-après :

### Exemple 1 : Obtention des souches DO et RE selon l'invention

La souche de Penicillium caseicolum CNCM I-1119 a été cultivée sous forme d'étalement direct des spores sur un milieu malt acide contenant 0,06 ppm de benlate.

Des thalles présentant de nombreux phénotypes différents sont apparus dès le 2ème jour. L'isolement sous la loupe du maximum d'individus a été pratiqué par repiquages simultanément sur un milieu minimum (contrôle de la prototrophie) et sur un milieu malt acide (multiplication de la souche).

A partir des isolements sur milieu malt acide, les individus caractérisés ont été testés pour leur activité inhibitrice des Mucor grâce à la technique des confrontations de souches sur milieu malt acide.

Les individus inhibant le Mucor, ayant une bonne croissance et une teinte du thalle blanche ont été retenus et leurs spores ont été directement remis en contact avec le benlate. Le produit de ces étalements a été soumis aux mêmes étapes que ci-dessus.

Après ces deux étapes successives de traitement par le benlate, on a retenu deux souches qui ont été dénommées arbitrairement DO et RE ; ces souches ont été à nouveau purifiées par isolement monospore et conservées sous azote liquide et sous huile de vaseline.

Elles ont ensuite été soumises aux différents tests permettant de mesurer les principales aptitudes technologiques :
- croissance sur milieu lactosérum,
- hauteur du mycélium sur milieu lactosérum,
- activité proléolytique sur milieu "skim milk",
- activité lipolytique sur milieu tributyrine.

Les protocoles et les milieux de culture, utilisés pour déterminer chaque aptitude, sont rassemblés dans la documentation technique des Laboratoires GRANDAY-ROGER : "Bulletin Technique 1 (1990) Aptitudes technologiques des souches ROGER® de Penicillium candidum". On notera que Penicillium candidum est la dénomination usuelle dans l'industrie fromagère de Penicillim caseicolum.

### 1 - La croissance

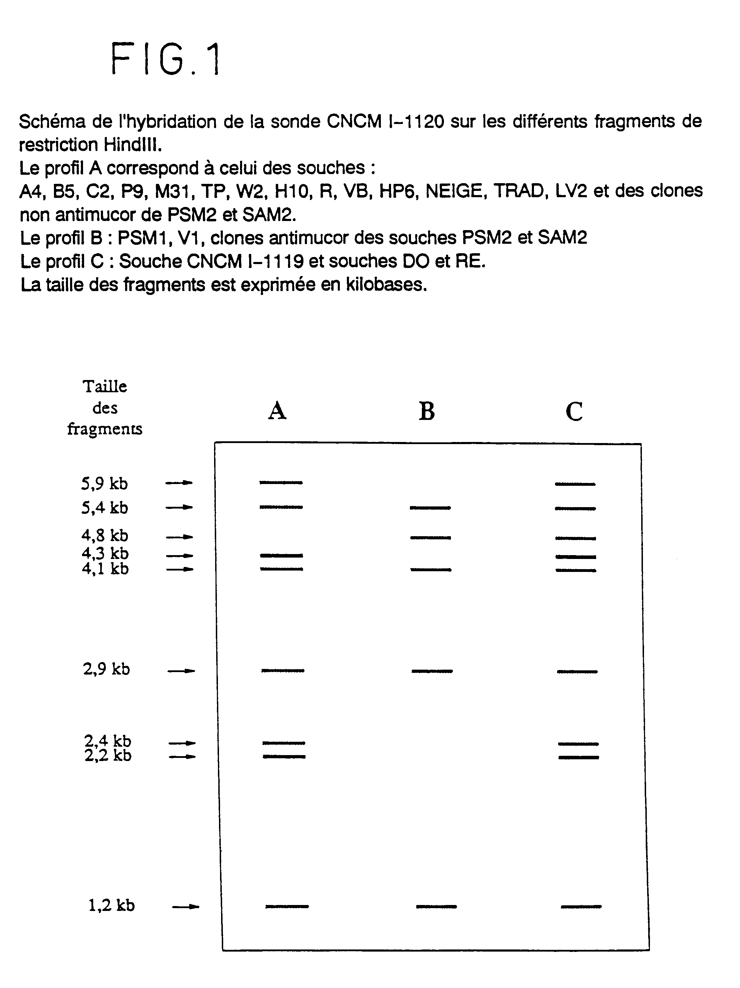

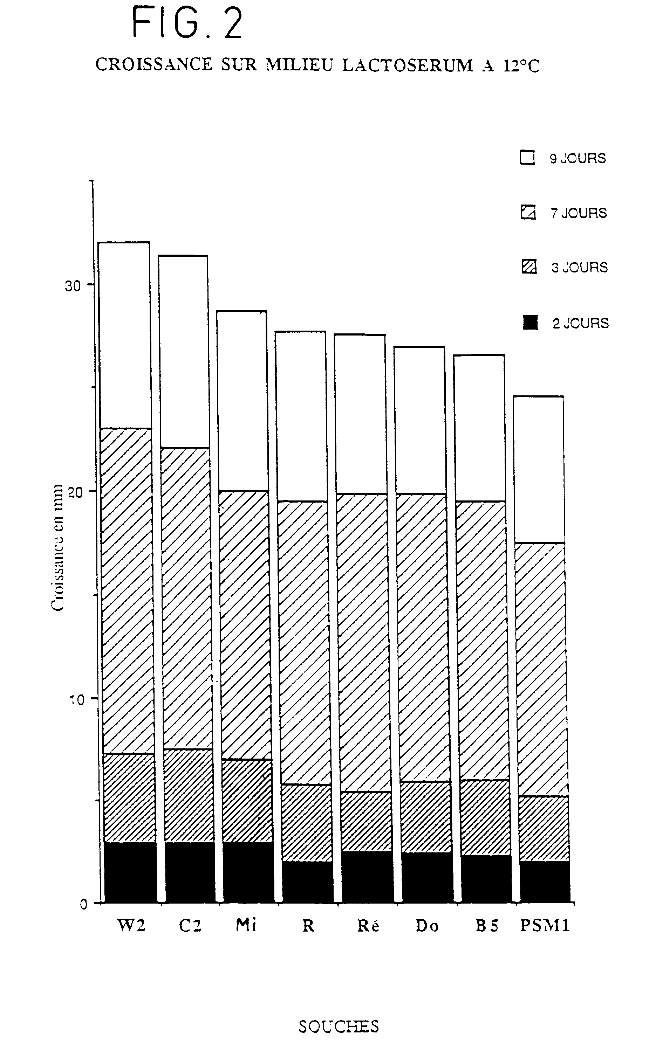

Sur un milieu lactosérum à base de sérum de fromagerie à 12°C, le suivi de la croissance montre que les nouvelles souches font partie de la catégorie des souches ayant une croissance rapide tout comme les souches R et B5 (figure 2).

### 2 - La hauteur du mycélium

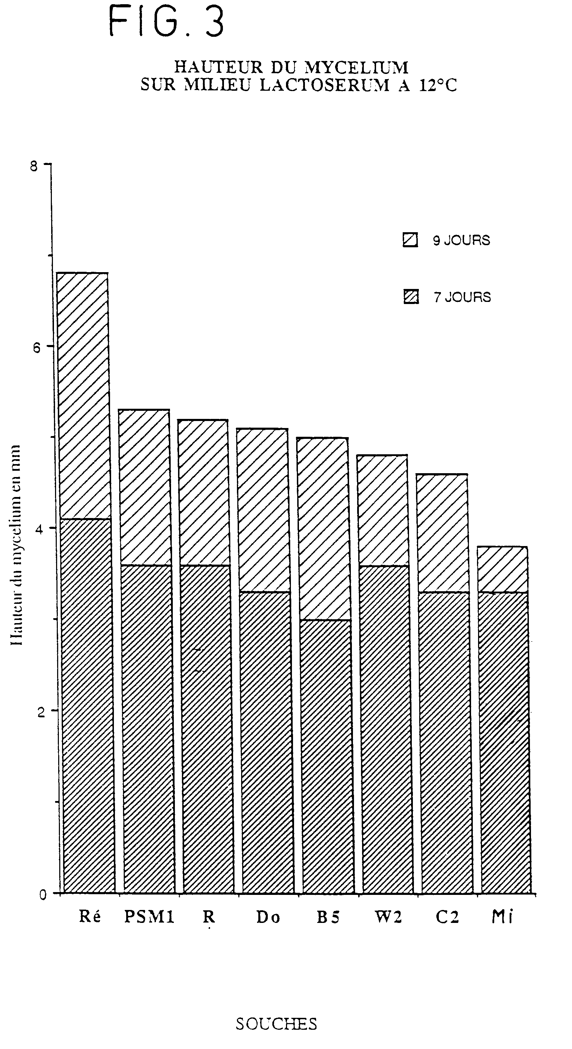

Sur le milieu lactosérum après 9 jours d'incubation à 12°C les souches présentent des hauteurs variables. Elles restent inférieures à 7 mm comme pour les autres souches (figure 3).

### 3 - La densité du mycélium

Elles présentent un mycélium plus aéré et peu dense. Ce critère n'est pas quantifiable, mais il est visible et reconnaissable par l'homme de l'art.

### 4 - Les activités protéolytiques et lipolytiques

Dans les conditions du test de caractérisation mis en place sur milieu "skim milk" (milieu lait écrémé), l'activité protéolytique globale des souches selon l'invention est comparable à celle des souches les plus couramment utilisées comme R, B5, W2 et C2.

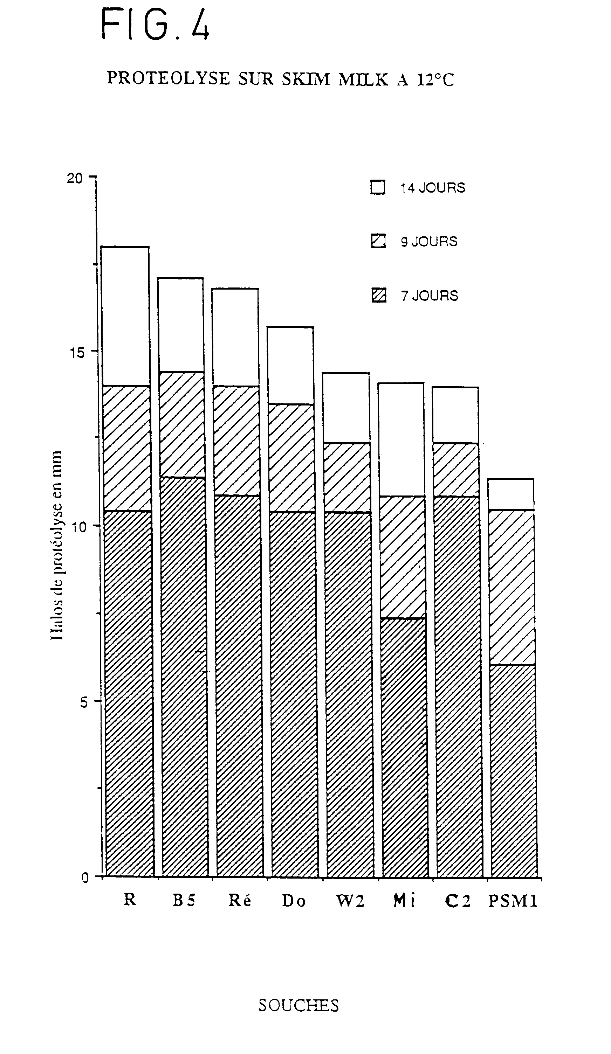

Elle est plus forte que celle de la souche "antimucor" PSM1 (figure 4).

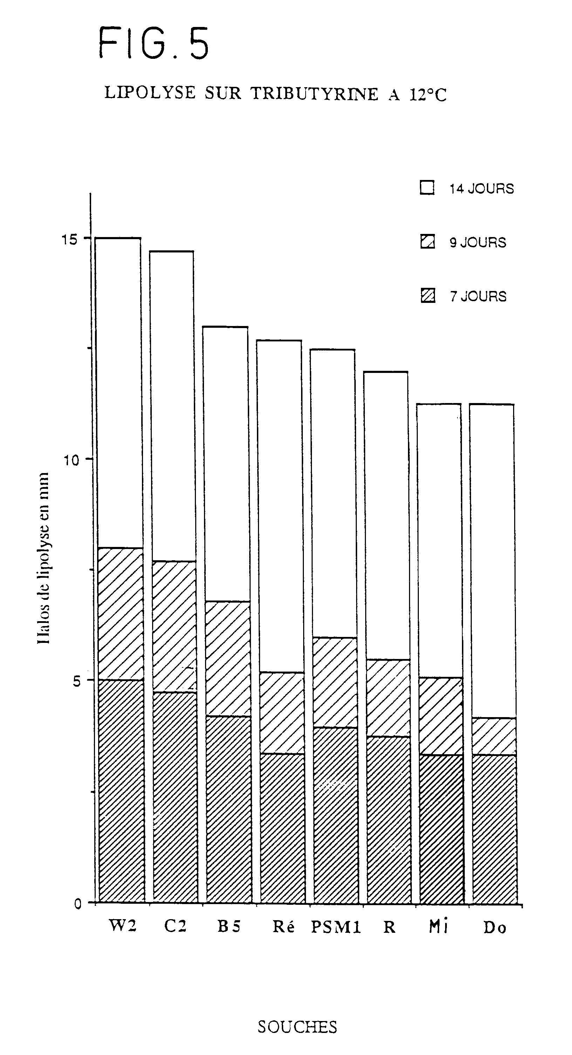

L'activité lipolytique à 12°C mesurée sur un milieu tributyrine est faible pour les deux souches DO et RE, (figure 5).

Les souches utilisées à titre de comparaison et dont les aptitudes sont également reportées sur les diagrammes des figures 2 à 5 sont les souches fromagères disponibles dans le commerce sous les dénominations W2, C2, R, B5 et PMS1 auprès des Laboratoires GRANDAY-ROGER. La souche "antimucor" PSM1 présente les nombreux défauts des souches "antimucor".

Les résultats obtenus figurent sur les figures 2 à 5 annexées comme indiqué ci-dessus.

On a également étudié le comportement des souches DO et RE sur tranches de fromages non affinés selon le protocole ci-après :

### 5 - Comportement sur tranches de fromage non affiné.

Des tranches de fromage non affinées ont été ensemencées par trempage dans des suspensions de spores des souches titrant 10⁷ spores/ml. Après incubation à 13°C en atmosphère à 90/95% d'humidité, on note les observations suivantes :
- l'apparition du mycélium des nouvelles souches est au moins aussi précoce que pour les souches habituelles (la souche R étant prise comme référence),
- le développement du mycélium est rapide et homogène à la surface du caillé,
- l'aspect de la zone de protéolyse après coupe transversale à 11 jours est normale,
- aucun défaut de couleur de ces souches n'est apparu au cours des essais.

L'ensemble du comportement de ces souches s'est donc avéré normal et parfaitement comparable aux souches habituellement mises en oeuvre en fromagerie.

### Exemple 2 : Caractérisation des souches DO et RE par hybridation avec la sonde d'ADN CNCM I-1120

Dans cet exemple on a utilisé les techniques décrites par SAMBROOK et al. dans l'ouvrage intitulé : MOLECULAR CLONING, Edition Cold Spring Harbor Laboratory Press, 1989.

200 ng de la sonde CNCM I-1120 radiomarquée sont utilisés pour chaque hybridation. Les hybridations sont menées 16 à 20 heures à 65°C dans un tampon d'hybridation. Deux lavages sont ensuite pratiqués à 65°C dans le tampon de faible stringence (2 X SSCet 0,1 SDS). La révélation de l'hybridation est effectuée après exposition 4 à 20 heures sur un film RPN8 de chez Amersham.

La sonde CNCM I-1120 a été hybridée sur la restriction enzymatique HindIII des ADN génomiques de différentes souches :
- un ensemble de souches du commerce : A4, B5, C2, P9,M31, TP, W2, H10, R, VB, HP6, NEIGE, TRAD, LV2, et les clones non "antimucor" des souches SAM2 et PSM2.
- les souches PSM1, V1 et les clones "antimucor" des souches PSM2 et SAM2
- les nouvelles souches DO et RE et la souche CNCM I-1119.

La sonde CNCM I-1120 s'hybride avec un nombre de fragments différents d'une souche à l'autre (figure n° 1). Ainsi, pour un groupe de souches nommé le groupe I, elle révèle huit fragments (profil A) :
1,2kb ; 2,2kb ; 2,4kb ; 2,9kb ; 4,1kb ; 4,3kb ; 5,4kb et 5,9kb.

Le groupe II présente cinq fragments (profil B) :
1,2kb ; 2,9kb ; 4,1kb ; 4,8kb et 5,4kb.

Les souches DO et RE présentent un profil différent des deux précédents avec 9 fragments de restriction et constitue un nouveau groupe III (profil C) :
1,2kb ; 2,2kb ; 2,4kb ; 2,9kb ; 4,1kb ; 4,3kb ; 4,8kb ; 5,4kb ; et 5,9kb.

La lecture de cet autoradiogramme fait ressortir que le profil des souches DO et RE est original puisque jamais observé chez les autres souches de Penicillium caseicolum étudiées.

En effet, ces deux souches présentent 9 fragments de restriction, (profil C) alors que les autres en présentent soit 8 soit 5. Les fragments 4,3kb, et 4,8kb sont présents ensemble dans la même souche.

### Exemple 3 : Obtention de la souche MI selon l'invention

La souche de Penicillium caseicolum CNCM I-1119 a été cultivée sous forme d'étalement direct des spores sur un milieu malt acide contenant 0,06 ppm de benlate.

Des thalles présentant de nombreux phénotypes différents sont apparus dès le 2ème jour. L'isolement sous la loupe du maximum d'individus a été pratiqué par repiquages simultanément sur un milieu minimum (contrôle de la prototrophie) et sur un milieu malt acide (multiplication de la souche).

A partir des isolements sur milieu malt acide, les individus caractérisés ont été testés pour leur activité inhibitrice des Mucor grâce à la technique des confrontations de souches sur milieu malt acide.

Les individus inhibant le Mucor, ayant une bonne croissance et une teinte du thalle blanche ont été retenus et leurs spores ont été directement remis en contact avec le benlate. Le produit de ces étalements a été soumis aux mêmes étapes que ci-dessus.

Après ces deux étapes successives de traitement par le benlate, on a retenu une souche qui a été dénommée arbitrairement MI ; cette souche a été à nouveau purifiée par isolement monospore et conservée sous azote liquide et sous huile de vaseline.

Elle a ensuite été soumise aux différents tests permettant de mesurer les principales aptitudes technologiques :
- croissance sur milieu lactosérum,
- hauteur du mycélium sur milieu lactosérum,
- activité proléolytique sur milieu "skim milk",
- activité lipolytique sur milieu tributyrine.

Les protocoles et les milieux de culture, utilisés pour déterminer chaque aptitude, sont rassemblés dans la documentation technique des Laboratoires GRANDAY-ROGER : "Bulletin Technique 1 (1990) Aptitudes technologiques des souches ROGER® de Penicillium candidum. On notera que Penicillium candidum est la dénomination usuelle dans l'industrie fromagère de Penicillim caseicolum.

### 1 - La croissance

Sur un milieu lactosérum à base de sérum de fromagerie à 12°C, le suivi de la croissance montre que la nouvelle souche fait partie de la catégorie des souches ayant une croissance rapide tout comme les souches R et B5 (figure 2).

### 2 - La hauteur du mycélium

Sur le milieu lactosérum après 9 jours d'incubation à 12°C la souche MI présente des hauteurs variables. Elles restent inférieures à 7 mm comme pour les autres souches (figure 3).

### 3 - La densité du mycélium

Elle présente un mycélium plus aéré et peu dense. Ce critère n'est pas quantifiable, mais il est visible et reconnaissable par l'homme de l'art.

### 4 - Les activités protéolytiques et lipolytiques

Dans les conditions du test de caractérisation mis en place sur milieu "skim milk" (milieu lait écrémé) l'activité protéolytique globale de la souche selon l'invention est comparable à celle des souches les plus couramment utilisées comme R, B5, W2 et C2.

Elle est plus forte que celle de la souche "antimucor" PSM1 (figure 4).

L'activité lipolytique à 12 °C mesurée sur un milieu tributyrine est faible pour la souche MI, (figure 5).

Les souches utilisées à titre de comparaison et dont les aptitudes sont également reportées sur les diagrammes des figures 2 à 5 sont les souches fromagères disponibles dans le commerce sous les dénominations W2, C2, R, B5 et PSM1 auprès des Laboratoires GRANDAY-ROGER. La souche "antimucor" PSM1 présente les nombreux défauts des souches "antimucor".

Les résultats obtenus figurent sur les figures 2 à 5 annexées comme indiqué ci-dessus.

On a également étudié le comportement de la souche MI sur tranches de fromages non affinés selon le protocole ci-après :

### 5 - Comportement sur tranches de fromage non affiné.

Des tranches de fromage non affinées ont été ensemencées par trempage dans des suspensions de spores des souches titrant 10⁷ spores /ml. Après incubation à 13 °C en atmosphère à 90/95 % d'humidité, on note les observations suivantes :
- l'apparition du mycélium de la nouvelle souche MI est au moins aussi précoce que pour les souches habituelles (la souche R étant prise comme référence),
- le développement du mycélium est rapide et homogène à la surface du caillé,
- l'aspect de la zone de protéolyse après coupe transversale à 11 jours est normale
- aucun défaut de couleur de cette souche n'est apparu au cours des essais.

L'ensemble du comportement de la nouvelle souche selon l'invention s'est donc avéré normal et parfaitement comparable aux souches habituellement mises en oeuvre en fromagerie.

### Exemple 4 : Caractérisation de la souche MI par hybridation avec la sonde d'ADN CNCM I-1120.

Dans cet exemple on a utilisé les techniques décrites par SAMBROOK et al. dans l'ouvrage intitulé : MOLECULAR CLONING, Edition Cold Spring Harbor Laboratory Press, 1989.

200 ng de la sonde CNCM I-1120 radiomarquée sont utilisés pour chaque hybridation. Les hybridations sont menées 16 à 20 heures à 65°C dans un tampon d'hybridation. Deux lavages sont ensuite pratiqués à 65°C dans un tampon de faible stringence (2XSSC et 0,1 SDS). La révélation de l'hybridation est effectuée après exposition 4 à 20 heures sur un film RPN8 de chez Amersham.

La sonde CNCM I-1120 a été hybridée sur la restriction enzymatique HindIII des ADN génomiques de différentes souches :
- un ensemble de souches du commerce : A4, B5, C2, P9,M31, TP, W2, H10, R, VB, HP6, NEIGE, TRAD, LV2, et les clones non "antimucor" des souches SAM2 et PSM2 (Groupe I)
- les souches PSM1, V1 et les clones "antimucor" des souches PSM2 et SAM2 (Groupe II)
- la nouvelle souche MI et la souche CNCM I-1120 (Groupe III).

La sonde CNCM I-1120 s'hybride avec un nombre de fragments différents d'une souche à l'autre (figure n° 1). Ainsi, pour un groupe de souches nommé le groupe I, elle révèle huit fragments (profil A) :
1,2kb; 2,2kb; 2,4kb; 2,9 kb; 4,1kb, 4,3kb; 5,4kb et 5,9kb.

Le groupe II présente cinq fragments (profil B) :
1,2kb; 2,9kb; 4,1kb; 4,8kb et 5,4kb.

La souche MI présente un profil du groupe II, mais à des différences quantitatives de certains fragments; on l'appelle profil B′. Ainsi, le fragment de 4,8 kb est moins dense, donc sous-représenté chez la souche MI, comparativement aux autres souches du Groupe II.

La souche CNCM I-1119 dont est issue la souche MI présente un profil de restriction différent des deux précédents contenant 9 fragments de restriction (profil C) : 1,2kb; 2,2kb; 2,4kb; 2,9kb; 4,1kb; 4,3kb; 4,8kb; 5,4kb; et 5,9kb.

## Revendications

**1 -** Souches fromagères de Penicillium caseicolum "antimucor", et présentant pour le couple enzyme de restriction Hind III-sonde d'ADN déposée dans des phages auprès de la Collection Nationale de Culture de Microorganismes sous le n° I-1120, un profil de restriction de type B′ ou de type C.

**2 -** Souches selon la revendication 1, caractérisées en ce qu'elles présentent le profil de restriction C.

**3 -** Souches selon la revendication 1, caractérisées en ce qu'elles présentent le profil de restriction B′.

**4 -** Procédé pour l'obtention de souches de Penicillium caseicolum "antimucor" et présentant des aptitudes fromagères, et des thalles de couleur blanche, caractérisé en ce qu'il consiste à :
1) étaler des spores de la souche CNCM I-1119 sur un milieu haploïdisant;
2) repiquer les colonies apparues sur un milieu minimum;
3) sélectionner parmi les souches obtenues celles qui présentent une activité "antimucor" et présentant des thalles de couleur blanche;
4) isoler parmi ces dernières celles qui présentent un profil de restriction pour le couple enzyme Hind III-sonde d'ADN déposée à la CNCM sous le n° I-1120, identique à l'un des profils B′ ou C.

**5 -** Procédé selon la revendication 4, caractérisé en ce que les étapes 1), 2) et 3) sont répétées une ou plusieurs fois sur les souches "antimucor" sélectionnées à l'étape 3).

**6 -** Utilisation de la souche CNCM I-1119 pour l'obtention de souches fromagères de Penicillium caseiculum "antimucor".
